# EUROPÄISCHE PATENTSCHRIFT

(11) **EP 0 861 829 B1**
(45) Veröffentlichungstag und Bekanntmachung des Hinweises auf die Patenterteilung: **28.04.2004**
(21) Anmeldenummer: 98102481.3
(22) Anmeldetag: 13.02.1998
(51) Int. Cl.: C07C 275/62, C07C 273/18, C08G 18/78

(54) **Verfahren zur Herstellung von Polyisocyanaten mit Biuretstruktur**
Process for the preparation of polyisocyanates having a biuret structure
Procédé de préparation de polyisocyanates à structure de biuret

(30) Priorität: 26.02.1997 DE 19707576
(43) Veröffentlichungstag der Anmeldung: 02.09.1998
(73) Patentinhaber: Bayer MaterialScience AG, 51368 Leverkusen (DE)
(72) Erfinder: Woynar, Helmut, Dr., 41540 Dormagen (DE); Schmidt, Manfred, Dr., 41540 Dormagen (DE); Grönen, Jürgen, Dr., 51491 Overath (DE)

(56) Entgegenhaltungen:
- EP-A- 0 003 505
- EP-A- 0 277 353

## Beschreibung

Die vorliegende Erfindung betrifft ein Verfahren zur Herstellung von Polyisocyanaten mit Biuretstruktur durch Umsetzung von überschüssigen Mengen an organischen Diisocyanaten mit aromatisch gebundenen Isocyanatgruppen mit organischen Diaminen bei erhöhten Temperaturen. Derartige Polyisocyanate können bei der Herstellung von Polyurethankunststoffen eingesetzt werden.

Die Herstellung von Polyisocyanaten mit Biuretstruktur durch direkte Umsetzung von überschüssigen Mengen an organischen Diisocyanaten mit organischen Diaminen bei erhöhten Temperaturen ist bekannt. So beschreibt beispielsweise die DE-OS 31 14 638 die Umsetzung von überschüßigen Mengen eines Gemisches aus 80 % 2,4- und 20 % 2,6-Diisocyanatotoluol mit Alkylphenylendiaminen, wobei die Reaktionspartner unter Zinkacetonylacetat-Katalyse innerhalb eines Zeitraumes von 3 Stunden bei 100°C umgesetzt werden. Diese Verfahrensweise, d.h. Herstellung von Biureten unter Katalysatorzugabe, benötigt zwar nur niedrige Temperaturen, in der Praxis möchte man die Verwendung von Katalysator jedoch nach Möglichkeit vermeiden, insbesondere, da zum Abstoppen der Reaktion anschließend der Katalysator mit Benzoylchlorid desaktiviert werden muß.

Einen technisch für aliphatische Isocyanate gut gangbaren Weg beschreibt die EP 0 003 505. Bei diesem Verfahren wird das Diamin in das vorgelegte Diisocyanat mit Hilfe einer Glattstrahldüse definierter Dimension unter Verwendung von hohen Drücken eingebracht. Nachteilig ist, daß bei Einsatz der beschriebenen Glattstrahldüse mit aromatischen Isocyanaten bei den in den Beispielen genannten Bedingungen Feststoffbildungen in Form von Harnstoffausfällungen nicht unterdrückt werden können. Aus diesem Grunde werden in Falle von aromatischen Isocyanaten in einer Vorstufe zuerst Harnstoffdispersion bei niedrigen Temperaturen bis 120°C gezielt hergestellt, um danach durch Aufheizen in die Biurete überführt zu werden.

Aufgabe der vorliegenden Erfindung war es daher, ein einstufiges Verfahren zur Herstellung von Polyisocyanaten mit Biuretstruktur auf Basis von Isocyanaten mit aromatisch gebundenen Isocyanatgruppen zur Verfügung zu stellen, ohne daß es dabei bei der Umsetzung der Isocyanate mit organischen Diaminen zu unerwünschten Feststoffbildungen kommt.

Wie jetzt überraschend gefunden wurde, ist es möglich, hochwertige Polyisocyanate mit Biuretstruktur auf Basis von aromatischen Diisocyanaten bzw. Diaminen herzustellen, wenn man die Ausgangsmaterialien bei oberhalb 180°C, vorzugsweise oberhalb 200°C liegenden Temperaturen, miteinander zur Reaktion bringt, wobei die Temperatur durch die Reaktionswärme weiter auf 220 - 270°C ansteigt. Dieser Befund ist äußerst überraschend, da man bislang der Ansicht war, daß oberhalb 220°C liegende Reaktionstemperaturen tunlichst zu vermeiden sind.

Gegenstand der Erfindung ist somit ein Verfahren zur Herstellung von Polyisocyanaten mit Biuretstruktur dadurch gekennzeichnet, daß man
a) organische Diisocyanate mit aromatisch gebundenen Isocyanatgruppen mit organischen Diaminen mit aromatisch gebundenen Aminogruppen in einem Molverhältnis von mindestens 8:1 in einer Mischkammer kontinuierlich zusammenführt und
b) bei einer Temperatur von oberhalb 180°C zur Reaktion bringt,
c) wobei die Verweilzeit der Reaktionspartner bzw. des Reaktionsgemisches in der Mischkammer als Vereinigung der Ausgangskomponenten maximal 60 Sekunden beträgt.

Ausgangsmaterialien für das erfindungsgemäße Verfahren sind organische Diisocyanate mit ausschließlich aromatisch gebundenen Isocyanatgruppen eines unter 300 liegenden Molekulargewichts, wie z.B. Toluylendiisocyanate, bevorzugt ein Gemisch aus 80 Gew.% 2,4-Toluylendiisocyanat und 20 Gew.% 2,6-Toluylendiisocyanat (TDI 80) oder ebenfalls bevorzugt TDI 100 oder TDI 65.

In gleicher Weise können auch Diphenylmethandiisocyanate, wie sie bei der technischen Herstellung anfallen, bevorzugt jedoch 4,4-Diisocyanatodiphenylmethan und dessen Isomere zur Anwendung kommen.

Weitere bevorzugte Ausgangsmaterialien für das erfindungsgemäße Verfahren sind organische Diamine mit aromatisch gebundenen Aminogruppen eines unter 300 liegenden Molekulargewichts. Bevorzugt sind 2,4-/2,6-Tolylendiamin bzw. Diphenylmethandiamine.

Bei der Durchführung des erfindungsgemäßen Verfahrens werden die Ausgangsdiisocyanate und die Diamine kontinuierlich in solchen Mengenverhältnissen zur Umsetzung gebracht, die einem Equivalenzverhältnis von Isocyanatgruppen zu Aminogruppen von mindestens 8:1, bevorzugt von 10:1 bis 20:1 entsprechen, wobei die Aminogruppen als monofunktionelle Gruppen in die Berechnung eingehen.

Erfindungswesentlich ist, daß die Ausgangsmaterialien sofort nach ihrer Durchmischung bei einer Temperatur von oberhalb 180°C, vorzugsweise von oberhalb 200°C, miteinander zur Reaktion gebracht werden. Diese hohen Reaktionstemperaturen zu Beginn der erfindungsgemäßen Umsetzung können durch Vorerhitzen des Diisocyanates auf Temperaturen von oberhalb 180°C, vorzugsweise von oberhalb 200°C erreicht werden. Die verwendeten Diamine werden üblicherweise bei Temperaturen von >100°C eingesetzt, um sie in flüssiger Form zu halten. In der Regel kann davon ausgegangen werden, daß sich das Reaktionsgemisch innerhalb weniger Sekunden wegen der hohen Wärmetönung auf eine Temperatur von 20 -70°C oberhalb der Ausgangstemperatur aufheizt.

Die in jedem Fall erforderliche Aufheizung der Diisocyanate muß wegen der bekannten Temperaturempfindlichkeit dieser Verbindungen innerhalb eines möglichst kurzen Zeitraums bewerkstelligt werden, vorzugsweise innerhalb eines Zeitraums von weniger als 30 Sekunden. Dies gelingt durch Verwendung von entsprechenden Wärmeaustauschaggregaten des Standes der Technik. Die Wärmetauscher können z.B. als Röhren-, Bündel- oder Plattenwärmeaustauscher ausgelegt sein. Sie können mit einem flüssigen Heizmedium, mit gespanntem Dampf oder mit direkter elektrischer Heizung betrieben werden. Besonders bevorzugt ist die Verwendung von solchen Wärmetauschern, die den Aufheizvorgang der Ausgangsdiisocyanate innerhalb eines Zeitraumes von weniger als 10 Sekunden gestatten.

Die kontinuierlichen Ströme der Reaktionspartner werden nach der beschriebenen Vorheizung in einer Mischkammer vereinigt. An die Leistungsfähigkeit der Mischkammer hinsichtlich einer intensiven Vermischung der Komponenten werden bei dem erfindungsgemäßen Verfahren keine besonderen Anforderungen gestellt.

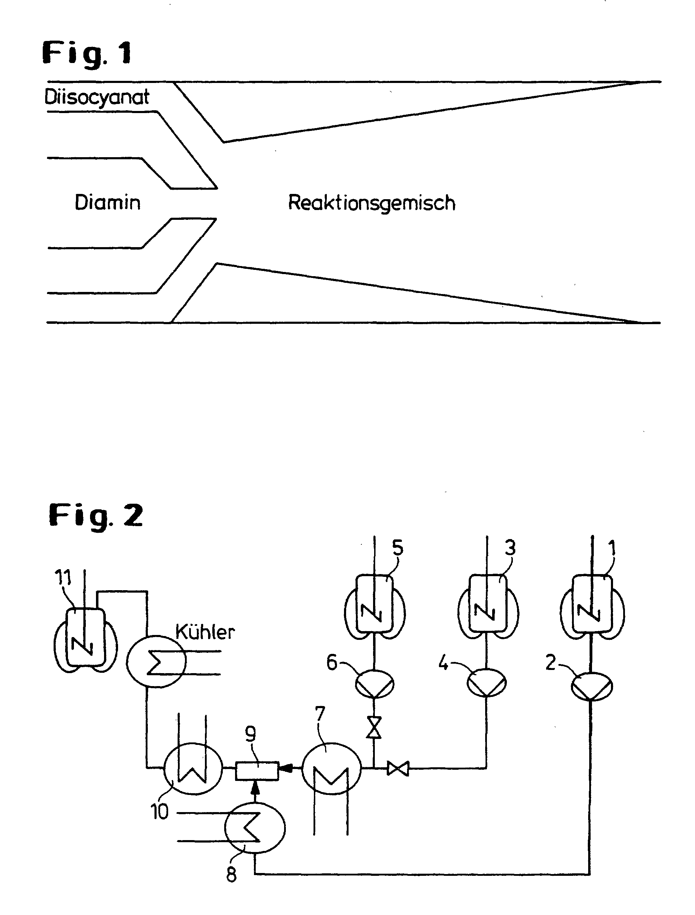

Die Eintrittsöffnungen der Komponenten in die Mischkammer sind vorzugsweise in Form von Düsen ausgebildet, damit die Zudosierung unter Überdruck erfolgen kann. Dadurch kann gewährleistet werden, daß das Reaktionsgemisch nicht in die Zuleitungen von Diisocyanat und Diamin gelangen kann. Hierfür werden die Querschnitte so gewählt, daß sich auf den Zuleitungen jeweils ein Druck von 1,5 bis 100 bar, vorzugsweise von 1,5 bis 40 bar aufbaut. Form und Anordnung der Düsen sowie hoher Druck sind für das Verfahren nicht erfindungswesentlich, da an die Mischleistung keine hohen Anforderungen gestellt werden. Dagegen ist durch die Geometrie der Mischkammer dafür Sorge zu tragen, daß der gesamte Materialtransport nach Möglichkeit rückströmungsfrei erfolgt, um somit lokale Überkonzentrationen des Amins und damit die Bildung von festen Polyharnstoffen zu vermeiden. Der prinzipielle Aufbau ist in Figur 1 dargestellt.

Das Volumen der Mischkammer und der nachgeschalteten, gegebenenfalls bereits gekühlten Verweilzeitstrecke sowie die Intensität der Kühlung in der nachgeschalteten Verweilzeitstrecke müssen so gewählt werden, daß die mittlere Verweilzeit des Reaktionsgemisches ab Vereinigung der Ausgangskomponenten bis Unterschreiten der Temperatur von 250°C maximal 60 Sekunden, vorzugsweise maximal 30 Sekunden und besonders bevorzugt maximal 10 Sekunden beträgt. Hierbei beträgt die mittlere Verweilzeit des Reaktionsgemisches bei den bevorzugten Temperaturen von oberhalb 270°C im allgemeinen höchstens 20 Sekunden, vorzugsweise höchstens 10 Sekunden und besonders bevorzugt höchstens 1 Sekunde.

Nach Durchlaufen der Mischkammer und der gegebenenfalls der Mischkammer nachgeschalteten Verweilzeitstrecke wird das Reaktionsgemisch kontinuierlich durch geeignete Wärmeaustauscher innerhalb von höchstens 10 Minuten, vorzugsweise höchstens 5 Minuten, stetig oder stufenweise auf eine Temperatur innerhalb des Temperaturbereichs von 120 bis 200°C, vorzugsweise 140 bis 160°C, abgekühlt. Wesentlich ist hierbei vor allem, daß das Reaktionsgemisch den Temperaturen von über 250°C nur innerhalb der obengenannten kurzen Zeiten ausgesetzt wird, wobei die Dauer der thermischen Nachbehandlung innerhalb weiter Grenzen schwanken kann. Im allgemeinen ist bei niedrigen Temperaturen innerhalb der zuletzt genannten Bereiche eine vergleichsweise lange, bei hohen Temperaturen eine vergleichsweise kurze thermische Nachbehandlung erforderlich. Anschließend wird mit Hilfe eines hochwirksamen Kühlers möglichst schnell auf eine Temperatur von <50°C abgekühlt, um die sonst als Nebenreaktion auftretende Dimerisierung, die zu unerwünschten Feststoffbildungen führen, zu unterdrücken.

Die nach dem erfindungsgemäßen Verfahren hergestellten, Biuretgruppen aufweisenden Polyisocyanate, stellen wertvolle Ausgangsmeterialien für die Herstellung von Zweikomponenten-Polyurethankunststoffen dar.

In den nachfolgenden Beispielen beziehen sich alle Prozentangaben auf Gewichtsprozente.

### Beispiel

- Fig. 1:: Prinzipieller Aufbau der Mischkammer
- Fig. 2:: mögliche Vorrichtung zur Durchführung des erfindungsgemäßen Verfahrens, wobei in Fig. 2 bedeuten
( 1) einen Rührwerksbehälter für Diisocyanat,
( 2) eine Förderpumpe für Diisocyanat,
( 3) einen Rührwerksbehälter für Diamin,
( 4) eine Förderpumpe für Diamin,
( 5) einen Rührwerksbehälter für Hilfslösungsmittel,
( 6) eine Förderpumpe für Hilfslösungsmittel,
( 7) einen Wärmeaustauscher zum Aufheizen von Diamin und Hilfslösungsmittel,
( 8) einen Wärmetauscher zum Aufheizen von Diisocyanat,
( 9) die Mischkammer,
(10) einen Wärmetauscher zum Abkühlen des Reaktionsgemisches und
(11) einen Rührbehälter für Verfahrensprodukt.

In den nachfolgenden Beispielen wurde die aus Fig. 2 ersichtliche Vorrichtung benutzt.

Das Hilfslösungsmittel (z.B. Xylol oder o-Dichlorbenzol aus (5)) wird lediglich am Anfang zum Einfahren der kontinuierlich betriebenen Apparatur verwendet und zusammen mit dem Diisocyanat in die Mischkammer geführt, um konstante Temperatur- und Druckbedingungen herzustellen, wodurch gewährleistet wird, daß keine Rückvermischung der Komponenten in den Zuleitungen auftreten kann. Die eigentliche Inbetriebnahme der Apparatur kann durch Umschalten vom Lösungsmittelstrom auf den Diaminstrom einfach und sicher vorgenommen werden. Vor dem Eintritt in die Mischkammer der Leitungen für Diisocyanat und Diamin sind düsenförmige Verengungen angebracht, um an dieser Stelle hohe Strömungsgeschwindigkeiten zu erzielen. Diese Düsen sind von ihrer Form her im Prinzip frei wählbar, da sie nicht die Aufgabe haben, Mischenergie in die Reaktionslösung einzubringen, sofern ein rückströmungsfreier Materialtransport gewährleistet ist.

Unmittelbar nach Austritt aus der Mischkammer wird das Reaktionsgemisch durch Wärmeaustauscher (10) innerhalb der in den Beispielen genannten Verweilzeiten auf das niedrige Temperaturniveau abgekühlt. Die thermische Nachbehandlung des Reaktionsproduktes erfolgt in dem mit kontinuierlichem Zu- und Ablauf versehenen Rührbehälter (11), könnte jedoch auch in einer Rührkesselkaskade oder einer entsprechend dimensionierten Verweilzeitstrecke erfolgen.

Als Rührwerksbehälter (1), (3), (5) und (11) wurden Glasgefäße eingesetzt, als Pumpen (2), (4) und (6) wurden Kolbendosierpumpen verwendet.

Als Wärmetauscher (7) und (8) wurden Doppelrohr-Wärmetauscher, die mit Öl oder Sattdampf als Wärmeträgermedium im Gegenstrom betrieben wurden, mit den folgenden Abmessungen eingesetzt.

| | (8) | (7) |
|---|---|---|
| Innenvolumen der Wärmeaustauscher | 22,8 cm³ | 0,4 cm³ |
| Wärmetauscherfläche | 415 cm² | 31,5 cm² |

Durch diese Abmessungen können die gewünschten kurzen Verweilzeiten bei hoher Temperatur erreicht werden.

Die Mischkammer (9) war als zylindrisches Rohr ausgebildet mit einer Düsenöffnung von 0,1 mm Durchmesser für das Diamin und einer Dimension von 5 cm Länge bei 2,5 mm Durchmesser. Der sich unmittelbar an die Mischkammer anschließende Wärmetauscher (10) war ebenfalls als Rohrwärmetauscher mit variablem Volumen ausgebildet und bot die Möglichkeit, verschiedene Abschnitte unterschiedlich zu temperieren. Die genauen Bedingungen werden in den einzelnen Beispielen gesondert aufgeführt.

### Beispiel 1 und 2:

Die Reaktionskomponenten wurden mit den in der Tabelle aufgeführten Förderraten in den Aufheizewärmetauschern auf die angegebene Temperatur aufgeheizt und anschließend in der Mischkammer zur Reaktion gebracht. Dabei erfolgte durch die Wärmetönung der Reaktion eine Aufheizung auf den genannten Wert.

Das Gemisch wird dann in den folgenden Wärmetauschern gezielt bis auf eine Temperatur von zuletzt ca. 160°C abgekühlt, danach möglichst schnell auf eine Temperatur <50°C. Man erhält Produkte mit NCO-Gehalten von 42 %, bzw. 37 % sowie dynamischen Viskositäten von 10 - 20, bzw. 100 - 150 mPas bei 25°C.

Als Diisocyanat kam ein Gemisch aus 80 Gew.-% 2,4-Toluylendiisocyanat und 20 Gew.-% 2,6-Toluylendiisocyanat zum Einsatz (Desmodur T80®), als Diamin ein entsprechendes Isomerengemisch der Toluylendiamine.

| | | **Beispiel 1** | **Beispiel 2** |
|---|---|---|---|
| **Diisocyanat:** | Mengenstrom (kg/h) | 91,7 | 75,3 |
| | Temperatur nach Aufheizen (°C) | 202 | 204 |
| **Diamin:** | Mengenstrom (kg/h) | 3,26 | 5,00 |
| | Temperatur nach Aufheizen (°C) | 146 | 156 |
| **Reaktionsgemisch:** | Temperatur nach ca. 1 s (°C) | 232 | 257 |
| | Temperatur nach ca. 3 s (°C) | 205 | 208 |
| | Temperatur nach ca. 10 s (°C) | 200 | 196 |
| | Temperatur nach ca. 60 s (°C) | 172 | 175 |
| | Temperatur nach ca 180 s(°C) | 160 | 158 |
| **Produkt:** | NCO-Gehalt (%) | 42 | 37 |
| | η_{25°C,} mPas | 10-20 | 100-150 |

### Beispiel 3 und 4:

In gleicher Weise wie in den Beispielen 1 und 2 wurde als Diisocyanatkomponente 4,4-Diisocyanatodiphenylmethan und 4,4-Diaminodiphenylmethan zur Reaktion gebracht. Man erhielt ein Produkt mit einem NCO-Gehalt von 28 % und einer Viskosität von 220 mPas bei 25°C bzw. einem NCO-Gehalt von 30,1 % und einer Viskosität von 59 mPas bei 25°C.

### Beispiel 5:

Bei Einsatz eines Gemisches aus ca. 45 % 4,4-Diisocyanatodiphenylmethan und ca. 55 % 2,4-Diisocyanatodiphenylmethan als Diisocyanatkomponente erhielt man in gleicher Weise ein Produkt mit einem NCO-Gehalt von 28,2 % und einer Viskosität 117 mPas.

| | | **Beispiel 3** | **Beispiel 4** | **Beispiel 5** |
|---|---|---|---|---|
| **Diisocyanat** | Mengenstrom (kg/h) | 104,8 | 96,8 | 74,9 |
| | Temperatur nach Aufheizen (°C) | 200 | 205 | 203 |
| **Diamin** | Mengenstrom (kg/h) | 5,01 | 3,22 | 3,53 |
| | Temperatur nach Aufheizen (°C) | 145 | 150 | 148 |
| **Reaktionsgemisch** | Temperatur nach ca. 1 s (°C) | 238 | 231 | 241 |
| | Temperatur nach ca. 3 s (°C) | 212 | 206 | 215 |
| | Temperatur nach ca. 10 s (°C) | 203 | 199 | 206 |
| | Temperatur nach ca. 60 s (°C) | 172 | 170 | 174 |
| | Temperatur nach ca. 180 s(°C) | 156 | 155 | 160 |
| **Produkt** | NCO-Gehalt (%) | 28,0 | 30,1 | 28,2 |
| | η_{25°C,} mPas | 220 | 59 | 117 |

## Patentansprüche

1. Verfahren zur Herstellung von Polyisocyanaten mit Biuretstruktur, **dadurch gekennzeichnet, daß** man
a) organische Diisocyanate mit aromatisch gebundenen Isocyanatgruppen mit organischen Diaminen mit aromatisch gebundenen Aminogruppen in einem Molverhältnis von mindestens 8:1 in einer Mischkammer kontinuierlich zusammenführt und
b) bei einer Temperatur von oberhalb 180°C zur Reaktion bringt,
c) wobei die Verweilzeit der Reaktionspartner bzw. des Reaktionsgemisches in der Mischkammer ab Vereinigung der Ausgangskomponenten maximal 60 Sekunden beträgt.

2. Verfahren nach Anspruch 1, **dadurch gekennzeichnet, daß** man die Diisocyanate mit den Diaminen in einem Molverhältnis von 10:1 bis 20:1 in der Mischkammer zusammenführt.

3. Verfahren nach einem der Ansprüche 1 bis 2, **dadurch gekennzeichnet, daß** man die Diisocyanate mit den Diaminen bei einer Temperatur im Bereich von 180 bis 270°C, vorzugsweise 200 bis 270°C, zur Reaktion bringt.

4. Verfahren nach einem der Ansprüche 1 bis 3, **dadurch gekennzeichnet, daß** man als organische Diisocyanate mit aromatisch gebundenen Isocyanatgruppen Toluylendiisocyanat einsetzt.

5. Verfahren nach einem der Ansprüche 1 bis 4, **dadurch gekennzeichnet, daß** man als organische Diisocyanate mit aromatisch gebundenen Isocyanatgruppen Diphenylmethandiisocyanat einsetzt.

6. Verfahren nach einem der Ansprüche 1 bis 5, **dadurch gekennzeichnet, daß** man als Diamine mit aromatisch gebundenen Aminogruppen solche mit einem unter 300 liegenden Molekulargewicht einsetzt.

## Claims

1. Process for the production of polyisocyanates having a biuret structure, **characterised in that**
a) organic diisocyanates having aromatically attached isocyanate groups are continuously combined in a mixing chamber with organic diamines having aromatically attached amino groups in a molar ratio of at least 8:1 and
b) are reacted at a temperature of above 180°C,
c) wherein the residence time of the reaction partners or reaction mixture in the mixing chamber from when the starting components are combined is at most 60 seconds.

2. Process according to claim 1, **characterised in that** the diisocyanates are combined with the diamines in the mixing chamber in a molar ratio of 10:1 to 20:1.

3. Process according to one of claims 1 to 2, **characterised in that** the diisocyanates are reacted with the diamines at a temperature in the range from 180 to 270°C, preferably of 200 to 270°C.

4. Process according to one of claims 1 to 3, **characterised in that** tolylene diisocyanate is used as the organic diisocyanates having aromatically attached isocyanate groups.

5. Process according to one of claims 1 to 4, **characterised in that** diphenyl methane diisocyanate is used as the organic diisocyanates having aromatically attached isocyanate groups.

6. Process according to one of claims 1 to 5, **characterised in that** the diamines having aromatically attached amino groups used are those having a molecular weight of below 300.

## Revendications

1. Procédé de préparation de polyisocyanates à structure de biuret, **caractérisé en ce que**
a) des diisocyanates organiques dotés de radicaux isocyanate liés aromatiquement sont amenés en continu avec des diamines organiques aux radicaux amino liés aromatiquement dans un rapport molaire d'au moins 8:1 dans une chambre de mélange et
b) mis en réaction à une température supérieure à 180°C;
c) le temps de séjour des partenaires de réaction ou du mélange réactif dans la chambre de mélange depuis l'association des composants de départ s'élevant à 60 secondes maximum.

2. Procédé selon la revendication 1, **caractérisé en ce que** les diisocyanates sont associés aux diamines dans un rapport molaire de 10:1 à 20:1 dans la chambre de mélange.

3. Procédé selon l'une des revendications 1 à 2, **caractérisé en ce que** les diisocyanates sont mis en réaction avec les diamines à une température de l'ordre de 180 à 270°C, de préférence de 200 à 270°C.

4. Procédé selon l'une des revendications 1 à 3, **caractérisé en ce que** du toluylènedlisocyanate est utilisé comme diisocyanate aromatique avec des radicaux isocyanate liés aromatiquement.

5. Procédé selon l'une des revendications 1 à 4, **caractérisé en ce que** du diphénylméthanediisocyanate est utilisé comme diisocyanate organique avec des radicaux isocyanate liés aromatiquement.

6. Procédé selon l'une des revendications 1 à 5, **caractérisé en ce que** des diamines à radicaux amino liés aromatiquement d'un point moléculaire inférieur à 300 sont utilisées.
